# EUROPÄISCHE PATENTSCHRIFT

(11) **EP 3 821 869 B1**
(45) Veröffentlichungstag und Bekanntmachung des Hinweises auf die Patenterteilung: **05.06.2024**
(21) Anmeldenummer: 20208059.4
(22) Anmeldetag: 17.11.2020
(51) Int. Cl.: A61H 9/00, A61B 5/145, A61B 5/00

(54) **SENSOREINHEIT FÜR EINE KOMPRESSIONSTHERAPIE UMFASSEND EINEN DRUCKSENSOR UND EINEN PULSOXIMETRISCHEN SAUERSTOFFSENSOR**
SENSOR UNIT FOR COMPRESSION THERAPY COMPRISING A PRESSURE SENSOR AND A PULSE OXIMETRIC OXYGEN SENSOR
UNITÉ DE CAPTEUR POUR UNE THÉRAPIE PAR COMPRESSION COMPRENANT UN CAPTEUR DE PRESSION ET UN CAPTEUR D'OXYGÈNE DE MESURE PAR OXYMÉTRIE DE POULS

(30) Priorität: 18.11.2019 DE 102019131056
(43) Veröffentlichungstag der Anmeldung: 19.05.2021
(73) Patentinhaber: Paul Hartmann AG, 89522 Heidenheim (DE)
(72) Erfinder: BAUER, Dr. Jochen, 89075 Ulm (DE); SMOLA, Prof. Dr., Hans, 66121 Saarbrücken (DE); ROCHE, Dr. Laurent, 89231 Neu-Ulm (DE); PEICHL, Frank, 73529 Schwäbisch Gmünd, Baden-Württemberg (DE); CROIZAT, Dr., Pierre, 89542 Herbrechtingen (DE); RAYER, Mathieu, 86199 Augsburg (DE); SPRENGER, Dennis, 90552 Röthenbach a. d. Pegnitz, Bayern (DE); MOOS, Gunnar, 81547 München, Bayern (DE); KITTLER, Jan-Philipp, 81675 München, Bayern (DE)
(74) Vertreter: Paul Hartmann AG Patents & Licensing

(56) Entgegenhaltungen:
- WO-A1-2019/072531
- WO-A1-2020/049291
- US-A1- 2019 254 906

## Beschreibung

Die Erfindung betrifft eine Sensoreinheit für eine Kompressionstherapie sowie einen Kit, ein Kompressionsmittel und ein Kompressionstherapiesystem umfassend die Sensoreinheit. Zudem betrifft die Erfindung ein Herstellungsverfahren für die Sensoreinheit.

Kompressionsbinden zur Behandlung der Veneninsuffizienz sind bekannt. Zur Behandlung der Veneninsuffizienz dürfen die Kompressionsbinden weder mit einem zu niedrigen noch mit einem zu hohen Druck angelegt werden. Bei einem zu niedrigen Anlegedruck ist die Behandlung gegebenenfalls wirkungslos. Bei einem zu hohen Anlegedruck werden die behandelten Köperteile gegebenenfalls nicht mehr richtig durchblutet und geschädigt. Um den richtigen Anlegedruck einzustellen, sind Kompressionsbinden mit mechanischen Druckindikatoren sowie mit elektronischen Drucksensoren bekannt.

Allerdings ist die Kontrolle des Anlegedrucks nicht in allen Fällen ausreichend, um eine komplikationslose Behandlung der Veneninsuffizienz zu gewährleisten. Zum Beispiel kann die alleinige Kontrolle des Anlegedrucks bei Patienten, welche gleichzeitig an einer Veneninsuffizienz und einer arteriellen Verschlusskrankheit (AVK) leiden, nicht ausreichend sein. Bei dieser Risikogruppe kann ein normalerweise unproblematischer Anlegedruck zu einer Minderperfusion der betroffenen Extremität und infolge dessen zu einem Untergang von Gewebe führen. Deshalb wird bei diesen Patienten aus Sicherheitsgründen oftmals keine Kompressionstherapie angewandt und deren Veneninsuffizienz unbehandelt gelassen, obwohl eine Kompressionstherapie unter Umständen möglich wäre und zu einer verbesserten Blutversorgung durch Abnahme der venösen Hypertension und somit zu einer Erhöhung der Druckdifferenz zwischen dem Blutdruck im arteriellen und venösen Kompartiment führen könnte.

Die US 2019/0254906 A1 offenbart ein System zur Bereitstellung einer Kompressionsbehandlung. Das System umfasst eine tragbare Kompressionsvorrichtung mit einem motorbetriebenen Kompressionsmechanismus, einem oder mehreren Sensoren, einer Steuereinheit und einem drahtlosen Kommunikationsmodul. Weiterhin umfasst das System ein Fernbedienungsgerät wie beispielsweise ein Smartphone, welches drahtlos mit dem Kommunikationsmodul der Kompressionsvorrichtung kommunizieren kann. Die US 906 offenbart auch ein Sensorpflaster.

Die WO 2019/072531 A1 offenbart ein Therapiesystem umfassend einen Kompressionsapparat, einen Prozessor und einen oder mehrere Sensoren. Die Sensoren können klebende Pads umfassen oder von einer Wundauflage getragen werden. Insbesondere soll der Kompressionsapparat die Wadenmuskulatur durch elektrische Stimulation aktivieren.

Die WO 2020/049291 A1 offenbart ein System umfassend eine optische Faseranordnung, die so konfiguriert ist, dass sie einen oder mehrere physiologische Parameter eines Individuums misst. Weiterhin umfasst das System einen Drucksensor, der so konfiguriert ist, dass er einen Kontaktdruck der optischen Faseranordnung auf das Individuum misst. Das System kann in eine Kompressionsbandage integriert sein. Zudem kann das System in der Form eines Pflasters ("patch") mit einem Pulsoximeter und einem Drucksensor ausgebildet sein.

Der vorliegenden Erfindung lag die Aufgabe zugrunde, die Sicherheit bei einer Kompressionstherapie zu verbessern. Diese Aufgabe wird mit einer Sensoreinheit nach Anspruch 1 und einem Herstellungsverfahren nach Anspruch 23 gelöst.

Erfindungsgemäß umfasst die Sensoreinheit für eine Kompressionstherapie ein Substrat, einen Drucksensor und einen pulsoximetrischen Sauerstoffsensor. Der Drucksensor und der Sauerstoffsensor sind auf dem Substrat angeordnet. Ferner können Messwerte des Drucksensors und des Sauerstoffsensors an einen Computer zur Überprüfung der Kompressionstherapie übertragen werden.

Die erfindungsgemäße Sensoreinheit ermöglicht es bei Verwendung in einer Kompressionstherapie zum einen, den auf das behandelte Körperteil des Patienten einwirkenden Druck zu überwachen. Zusätzlich ermöglicht es die Sensoreinheit, die Sauerstoffsättigung des arteriellen Blutes in dem behandelten Körperteil des Patienten zu überwachen. Mit der ersten Maßnahme - der Drucküberwachung - kann insbesondere ein akutes Versagen der Kompressionstherapie bei einem zu niedrigen Druck oder eine akute Gefahrensituation infolge der Kompressionstherapie bei einem zu hohen Druck erfasst werden. Die zweite Maßnahme - die Überwachung der Sauerstoffsättigung - dient insbesondere dazu, eine Mangeldurchblutung des behandelten Körperteils infolge einer für den Patienten zu starken Kompression zu erfassen. Eine solche Mangeldurchblutung kann bei den zuvor genannten Risikopatienten bereits dann auftreten, wenn der Druck den oben erwähnten Druckbereich noch gar nicht überschritten hat. Durch die gleichzeitige Überwachung von Druck und Sauerstoffsättigung wird jedenfalls die Sicherheit der Kompressionstherapie erhöht. Deswegen kann die Kompressionstherapie unter Verwendung der hier vorgeschlagenen Sensoreinheit einem größeren Patientenkreis zugänglich gemacht werden.

Vorteilhaft an der vorliegenden Erfindung ist zudem, dass der Drucksensor und der Sauerstoffsensor eine Einheit bilden. Dadurch kann die Handhabung der Sensoren verbessert und an derselben Körperstelle sowohl der Druck als auch die Sauerstoffsättigung gemessen werden. Weitere Vorteile der Erfindung ergeben sich aus den nachfolgend beschriebenen bevorzugten Ausführungsformen.

Bei dem Substrat der Sensoreinheit handelt es sich um eine Leiterplatte, insbesondere um eine flexible Leiterplatte. Die Befestigung der Sensoreinheit am Körper des Patienten beziehungsweise an einem Kompressionsmittel kann erleichtert werden, indem die Leiterplatte flexibel ausgebildet ist. Die flexible Leiterplatte verbessert insbesondere auch den Tragekomfort der Sensoreinheit. Bevorzugt handelt es sich bei der flexiblen Leiterplatte um einen Polymerfilm, auf dem elektronische Komponenten aufgebracht, insbesondere aufgedruckt, sind.

Bei dem Drucksensor kann es sich um einen Dehnungsmessstreifen, einen induktiven Drucksensor oder einen piezoelektrischen Drucksensor handeln. Bevorzugt wird aus dieser Gruppe der piezoelektrische Drucksensor.

Typischerweise umfasst der Sauerstoffsensor eine Lichtquelle für rotes Licht, eine Lichtquelle für infrarotes Licht und einen Photodetektor. Die Lichtquellen sind vorzugsweise Leuchtdioden (light-emitting diode, LED). Der Photodetektor ist vorzugsweise eine Photodiode. Ein solcher Sauerstoffsensor wird unter der Bezeichnung BIOFY^{®} SFH7060 von Osram Opto Semiconductors (Regensburg, Deutschland) vertrieben. Die Lichtquellen können dafür verwendet werden, um Licht der Wellenlängen 660 nm (rotes Licht) und 940 nm (infrarotes Licht) zu erzeugen. Der Photodetektor kann das durch das Gewebe hindurchtretende beziehungsweise von dem Gewebe reflektierte Licht erfassen, woraufhin von dem Computer über im Stand der Technik an sich bekannte Verfahren die Sauerstoffsättigung des arteriellen Blutes berechnet werden kann. Entsprechend kann der anspruchsgemäße Messwert des Sauerstoffsensors Messwerte des Photodetektors im Hinblick auf das transmittierte beziehungsweise reflektierte rote Licht sowie Messwerte des Photodetektors im Hinblick auf das transmittierte beziehungsweise reflektierte infrarote Licht umfassen.

Weiterhin handelt es sich bei dem Sauerstoffsensor vorzugsweise um einen reflexions-pulsoximetrischen Sauerstoffsensor. Das bedeutet, dass der pulsoximetrische Sauerstoffsensor vorzugsweise das Prinzip der Reflexionsoximetrie nutzt. Damit wird die Anordnung des Sauerstoffsensors am Körper des Patienten deutlich vereinfacht. Die zuvor genannten Bestandteile des Sauerstoffsensors (Lichtquellen und Detektor) müssen dann nämlich nicht wie bei der alternativen Transmissionsoximetrie an sich gegenüberliegenden Körperstellen angeordnet werden, sondern können an derselben Körperstelle platziert werden. Die erfindungsgemäß vorgesehene Sensoreinheit könnte aus technischer Sicht mit einem transmissions-pulsoximetrischen Sauerstoffsensor nur schwierig umgesetzt werden, so dass diese Variante weniger bevorzugt ist.

Vorzugsweise handelt es sich bei dem Computer um ein mobiles Gerät. Als mobiles Gerät kommt für die Erfindung insbesondere ein Smartphone oder ein Tablet in Betracht. Dadurch kann die Anzahl der elektronischen Komponenten auf der Sensoreinheit minimiert werden, weil auf ein bereits vorhandenes, leistungsstarkes Gerät des Patienten oder des Pflegepersonals für die Datenauswertung und Datenanzeige zurückgegriffen werden kann. Zudem kann damit die Handhabung und der Tragekomfort eines die Sensoreinheit umfassenden Kompressionstherapiesystems verbessert werden.

In einer einfachen Ausgestaltung der Erfindung werden die Messwerte des Drucksensors und des Sauerstoffsensors an den Computer kabelgebunden übertragen. Viel vorteilhafter ist es aber, wenn die Übertragung der Messwerte des Drucksensors und des Sauerstoffsensors an den Computer kabellos erfolgt, wobei die Übertragung vorzugsweise über eine Nahfeldkommunikation (near field communication, NFC), Bluetooth oder ein drahtloses lokales Netzwerk (wireless local area network, WLAN) stattfindet. Dadurch können die Messwerte an einen beliebigen Ort übertragen und die Handhabung sowie der Tragekomfort der Sensoreinheit verbessert werden. Für den kabellosen Datenaustausch kann die Sensoreinheit einen NFC-Sendeempfänger, einen Bluetooth-Sendeempfänger und/oder einen WLAN-Sendeempfänger umfassen. Typischerweise umfasst der Computer dann auch einen solchen NFC-Sendeempfänger, Bluetooth-Sendeempfänger und/oder WLAN-Sendeempfänger.

Um die Datensicherheit zu verbessern, wird außerdem vorgeschlagen, dass die Übertragung der Messwerte des Drucksensors und des Sauerstoffsensors an den Computer verschlüsselt erfolgt. Die Verschlüsselung kann zum Beispiel von einer von der Sensoreinheit umfassten Datenerfassungseinheit durchgeführt werden. Die Entschlüsselung der Messwerte kann von dem Computer durchgeführt werden.

Die bereits erwähnte Datenerfassungseinheit ist vorrangig dafür ausgebildet die Messwerte von dem Drucksensor und dem Sauerstoffsensor zu erfassen und an den Computer zu übertragen. Ebenso wie die Sensoren kann die Datenerfassungseinheit auf dem Substrat der Sensoreinheit angeordnet sein. Sie kann aber auch mit einem Konnektor, beispielsweise einem Kabel, mit dem Substrat der Sensoreinheit verbunden sein. Die Datenerfassungseinheit kann einen Datenträger und den Sendeempfänger der Sensoreinheit (NFC-, Bluetooth- und/oder WLAN-Sendeempfänger) umfassen. Außerdem kann die Datenerfassungseinheit einen Microcontroller, einen Vorverstärker und einen Analog-DigitalWandler umfassen. Somit kann die Datenerfassungseinheit für sich gesehen gleichfalls einen Computer darstellen.

Ein weiterer Vorteil ergibt sich, wenn die Sensoreinheit eine Energiequelle, vorzugsweise eine Batterie, für den Drucksensor und den Sauerstoffsensor umfasst. Durch die Energiequelle kann eine permanente Aktivierung der Sensoren, also ein kontinuierliches Monitoring der Druck- und Sauerstoffverhältnisse, ermöglicht werden. Die Energiequelle kann auf dem Substrat der Sensoreinheit angeordnet sein. Denkbar ist allerdings auch, dass die Sensoreinheit keine Energiequelle für den Drucksensor und den Sauerstoffsensor umfasst. Dadurch kann die Sensoreinheit vereinfacht und deren Tragekomfort erhöht werden. Die Energieversorgung der Sensoren in dieser speziellen Ausgestaltung der Erfindung kann zum Beispiel mittels NFC-Funksignalen des verwendeten Smartphones erfolgen.

Gemäß einer besonders bevorzugten Ausführungsform der Erfindung ist die Sensoreinheit adhäsiv ausgebildet. Dafür umfasst die Sensoreinheit vorzugsweise eine adhäsive Rückschicht, wobei die Sensoreinheit dann in der Art eines Pflasters ausgestaltet sein kann. Diese Ausführungsform hat den besonderen Vorteil, dass sich die Sensoreinheit leicht und ohne zusätzliche Hilfsmittel (wie etwa einem Heftpflaster) am Körper des Patienten anbringen lässt.

Gegenstand der vorliegenden Erfindung ist auch ein Kit für eine Kompressionstherapie. Der Kit umfasst ein Kompressionsmittel, eine oder mehrere erfindungsgemäße Sensoreinheiten in allen ihren Ausführungsformen, optional ein oder mehrere Heftpflaster und optional eine Gebrauchsanweisung. Das Heftpflaster ist dabei zur Befestigung der Sensoreinheit am Körper des Patienten vorgesehen und vorteilhaft in dem Kit enthalten, falls die Sensoreinheit nicht adhäsiv ausgebildet ist. Mit dem Kit können dem Patienten alle Verbandskomponenten für die Kompressionstherapie zusammen mit Anwendungshinweisen in leicht handhabbarer und übersichtlicher Art und Weise zur Verfügung gestellt werden.

Anstatt das Kompressionsmittel und die Sensoreinheit wie in dem zuvor genannten Kit vorgesehen losgelöst voneinander bereitzustellen, kann die Sensoreinheit auch herstellerseitig mit einem Kompressionsmittel verbunden bereitgestellt werden. Entsprechend wird vorliegend auch ein Kompressionsmittel umfassend eine oder mehrere erfindungsgemäße Sensoreinheiten in allen ihren Ausführungsformen beansprucht. Dadurch erfolgt die Befestigung der Sensoreinheit am Körper des Patienten gleichzeitig mit dem Anlegen des Kompressionsmittels. Es entfällt also ein Arbeitsschritt für den Anwender (nämlich das separate Anbringen der Sensoreinheit an der Messstelle), was die Vorbereitung der Kompressionstherapie beschleunigen und vereinfachen kann. Gegebenenfalls ist der Anwender dann jedoch nicht mehr vollkommen frei in seiner Wahl, an welcher Stelle die Sensoreinheit zur Erhebung der Messwerte platziert werden soll, da sich durch die herstellerseitige Integrierung der Sensoreinheit in das Kompressionsmittel eine bestimmte Sensoranordnung am Körper des Patienten automatisch ergibt. Eine Integrierung der Sensoreinheit in das Kompressionsmittel ist dennoch insbesondere dann sinnvoll, wenn als Drucksensor ein Dehnungsmessstreifen verwendet wird.

Ein weiterer Gegenstand der Erfindung betrifft ein System für eine Kompressionstherapie. Dieses System umfasst ein Kompressionsmittel, eine erfindungsgemäße Sensoreinheit in allen ihren Ausführungsformen und einen Computer. Messwerte des von der Sensoreinheit umfassten Drucksensors und pulsoximetrischen Sauerstoffsensors können an den Computer übertragen werden. Der Computer umfasst Mittel zur Ausführung eines Verfahrens zur Überprüfung der Kompressionstherapie, wobei das Verfahren die folgenden Schritte umfasst:
i. Empfangen und Prüfen eines Messwertes des Drucksensors während der Kompressionstherapie,
   wobei geprüft wird, ob der Messwert des Drucksensors außerhalb eines vorgegebenen Druckbereiches liegt, und
   wobei vorzugsweise ein Alarm ausgegeben wird, wenn die Prüfung ergibt, dass der Messwert des Drucksensors außerhalb des vorgegebenen Druckbereiches liegt,
ii. Empfangen und Prüfen eines Messwertes des Sauerstoffsensors während der Kompressionstherapie,
   wobei geprüft wird, ob der Messwert des Sauerstoffsensors eine vorgegebene Untergrenze für eine Sauerstoffsättigung unterschreitet, und
   wobei vorzugsweise ein Alarm ausgegeben wird, wenn die Prüfung ergibt, dass der Messwert des Sauerstoffsensors die vorgegebene Untergrenze für die Sauerstoffsättigung unterschreitet.

Mit dem obigen Verfahren zur Überprüfung der Kompressionstherapie wird eine konkrete Möglichkeit angegeben, wie die von der Sensoreinheit erfassten Messdaten effektiv und im Sinne einer sicheren Kompressionstherapie ausgewertet werden können.

Bei den zuvor genannten Mitteln des Computers handelt es sich insbesondere um einen Prozessor und ein Computerprogramm.

Gegebenenfalls kann eine Weiterverarbeitung der Messwerte des Drucksensors und insbesondere des Sauerstoffsensors erforderlich sein, damit der Abgleich mit den im vorliegenden Dokument genannten Referenzbereichen und Grenzwerten erfolgen kann. Eine solche Weiterverarbeitung der Messwerte kann von dem Computer im Rahmen der entsprechenden Prüfungen durchgeführt werden. Zudem kann es für die Berechnung einer arteriellen Sauerstoffsättigung sowie eines Pulsschlages notwendig sein, dass mehrere Messwerte von dem Sauerstoffsensor in einer zeitlichen Abfolge erfasst und von dem Computer weiterverarbeitet werden. Daher kann der Messwert aus dem obigen Verfahrensschritt ii. auch mehrere Messwerte umfassen, welche in einer zeitlichen Abfolge von dem Sauerstoffsensor erfasst und von dem Computer zu einer gemessenen Sauerstoffsättigung (oder auch eines gemessenen Pulsschlages) weiterverarbeitet werden. Es ist auch möglich, dass die Sensoreinheit (beispielsweise deren Datenerfassungseinheit) den Computer des Kompressionstherapiesystems bei der Prüfung der Messwerte des Drucksensors und des Sauerstoffsensors unterstützt. Zum Beispiel könnte die Sensoreinheit die eben beschriebene Weiterverarbeitung der Messwerte des Drucksensors und des Sauerstoffsensors durchführen.

Das zuvor bereits mehrfach erwähnte Kompressionsmittel kann eine Kompressionsbinde, ein Kompressionsschlauch oder ein Kompressionsstrumpf sein. Die Kompressionsbinde hat den Vorteil, dass sie an verschiedene Körperteile angelegt werden kann. Der Kompressionsschlauch und der Kompressionsstrumpf können leicht und schnell angelegt werden. Kompressionsbinden sind im Handel erhältlich, zum Beispiel von der Patentanmelderin Paul Hartmann AG (Heidenheim, Deutschland) unter dem Markennamen Pütter^{®}. Es können auch mehrere der zuvor genannten Kompressionsmittel miteinander kombiniert werden. Zum Beispiel kann eine Kompressionsbinde mit einem Kompressionsstrumpf kombiniert eingesetzt werden.

Gemäß einer bevorzugten Ausführungsform umfasst das Kompressionstherapiesystem mehrere Sensoreinheiten und damit auch mehrere Druck- und Sauerstoffsensoren. Dadurch können an verschiedenen Stellen unter dem Kompressionsmittel Druck und Sauerstoffsättigung bestimmt werden. Die Genauigkeit des Systems wird erhöht. Wenn das Kompressionstherapiesystem gemäß dieser bevorzugten Ausführungsform mehrere Drucksensoren und mehrere pulsoximetrische Sauerstoffsensoren umfasst, kann jeder der zusätzlichen Sensoren insofern dies nicht bereits explizit vorgesehen ist auch ein Teil der nachfolgend beschriebenen Ausführungsformen sein. Wenn also in einer nachfolgend genannten Ausführungsform des Kompressionstherapiesystems von einem Drucksensor oder einem Sauerstoffsensor gesprochen wird, können bei Kombination dieser nachfolgenden Ausführungsform mit der vorliegenden Ausführungsform auch mehrere Drucksensoren oder mehrere Sauerstoffsensoren gemeint sein.

Bei Vorhandensein von mehreren Drucksensoren kann in Schritt i. des Verfahrens zur Überprüfung der Kompressionstherapie von jedem Drucksensor jeweils ein Messwert empfangen werden, wobei die Messwerte der Drucksensoren zusammengefasst werden, um einen einzelnen, zusammengefassten Drucksensor-Messwert zu bilden. Der zusammengefasste Drucksensor-Messwert wird dann dahingehend geprüft, ob er außerhalb des vorgegebenen Druckbereiches liegt. Vorzugsweise wird der Alarm ausgegeben, wenn die Prüfung ergibt, dass der zusammengefasste Drucksensor-Messwert außerhalb des vorgegebenen Druckbereiches liegt. Bevorzugt ist der zusammengefasste Drucksensor-Messwert ein Mittelwert.

Schritt i. des Verfahrens zur Überprüfung der Kompressionstherapie könnte bei Vorhandensein von mehreren Drucksensoren demnach lauten:
Empfangen und Prüfen jeweils eines Messwertes von jedem (der mehreren) Drucksensor(en) während der Kompressionstherapie,
wobei die Messwerte der Drucksensoren zusammengefasst werden, um einen einzelnen, zusammengefassten Drucksensor-Messwert zu bilden,
wobei geprüft wird, ob der zusammengefasste Drucksensor-Messwert außerhalb eines vorgegebenen Druckbereiches liegt, und
wobei vorzugsweise ein Alarm ausgegeben wird, wenn die Prüfung ergibt, dass der zusammengefasste Drucksensor-Messwert außerhalb des vorgegebenen Druckbereiches liegt.

Bei Vorhandensein von mehreren Sauerstoffsensoren kann in Schritt ii. des Verfahrens zur Überprüfung der Kompressionstherapie von jedem Sauerstoffsensor jeweils ein Messwert empfangen werden, wobei die Messwerte der Sauerstoffsensoren zusammengefasst werden, um einen einzelnen, zusammengefassten Sauerstoffsensor-Messwert zu bilden. Der zusammengefasste Sauerstoffsensor-Messwert wird dann dahingehend geprüft, ob er die vorgegebene Untergrenze für die Sauerstoffsättigung unterschreitet. Vorzugsweise wird der Alarm ausgegeben, wenn die Prüfung ergibt, dass der zusammengefasste Sauerstoffsensor-Messwert die vorgegebene Untergrenze unterschreitet. Bevorzugt ist der zusammengefasste Sauerstoffsensor-Messwert ein Mittelwert.

Schritt ii. des Verfahrens zur Überprüfung der Kompressionstherapie könnte bei Vorhandensein von mehreren Sauerstoffsensoren demnach lauten:
Empfangen und Prüfen jeweils eines Messwertes von jedem (der mehreren) Sauerstoffsensor(en) während der Kompressionstherapie,
wobei die Messwerte der Sauerstoffsensoren zusammengefasst werden, um einen einzelnen, zusammengefassten Sauerstoffsensor-Messwert zu bilden,
wobei geprüft wird, ob der zusammengefasste Sauerstoffsensor-Messwert eine vorgegebene Untergrenze für eine Sauerstoffsättigung unterschreitet, und
wobei vorzugsweise ein Alarm ausgegeben wird, wenn die Prüfung ergibt, dass der zusammengefasste Sauerstoffsensor-Messwert die vorgegebene Untergrenze für die Sauerstoffsättigung unterschreitet.

Alternativ kann bei Vorhandensein von mehreren Druck- und Sauerstoffsensoren von jedem Druck- und Sauerstoffsensor jeweils ein Messwert empfangen und separat geprüft werden. Damit können die Messstellen mit den Sensoreinheiten einzeln im Hinblick auf Druck und Sauerstoffsättigung überwacht werden, was vorliegend als besonders vorteilhaft angesehen wird.

Zudem wird bevorzugt, dass das Empfangen und Prüfen der Messwerte des Drucksensors und des Sauerstoffsensors unmittelbar nachdem die Messwerte von den Sensoren erfasst wurden erfolgt. Dadurch kann der Benutzer des Systems über den aktuellen Zustand der Kompressionstherapie informiert werden. Ebenso wird bevorzugt, dass das Empfangen und Prüfen des Messwertes des Drucksensors und das Empfangen und Prüfen des Messwertes des Sauerstoffsensors gleichzeitig erfolgen. Dadurch kann der Benutzer des Systems umfassend über den Zustand der Kompressionstherapie informiert werden.

Weiterhin kann das Empfangen und Prüfen der Messwerte des Drucksensors und des Sauerstoffsensors automatisch und kontinuierlich nach Beginn der Kompressionstherapie erfolgen oder zumindest automatisch in regelmäßigen Abständen nach Beginn der Kompressionstherapie erfolgen. Die Überprüfung der Messwerte kann dann nicht vergessen werden, was die Sicherheit des Systems erhöht. Alternativ dazu kann das Empfangen und Prüfen der Messwerte des Drucksensors und des Sauerstoffsensors ausschließlich auf Befehl eines Benutzers hin erfolgen. Dadurch kann das System vereinfacht werden, weil dann zum Beispiel auf eine Energiequelle für die Sensoren auf der Sensoreinheit verzichtet werden kann.

Der von dem Computer zur Überprüfung des Druck-Messwertes herangezogene Druckbereich hat eine Untergrenze und eine Obergrenze. Die Untergrenze des Druckbereiches kann einen Wert von 1 mmHg bis 20 mmHg, bevorzugt 10 mmHg bis 20 mmHg und besonders bevorzugt 15 mmHg bis 20 mmHg aufweisen. Die Obergrenze des Druckbereiches kann einen Wert von 40 mmHg bis 200 mmHg, bevorzugt 40 mmHg bis 120 mmHg, mehr bevorzugt 40 mmHg bis 100 mmHg, noch mehr bevorzugt 40 mmHg bis 80 mmHg und besonders bevorzugt 40 mmHg bis 60 mmHg aufweisen. Insbesondere weist die Untergrenze einen Wert von 20 mmHg und die Obergrenze einen Wert von 40 mmHg auf, so dass sich ein Druckbereich von 20 mmHg bis 40 mmHg ergibt. Ein Alarm würde dann ausgegeben werden, wenn der gemessene Druck außerhalb des Druckbereiches von 20 mmHg bis 40 mmHg liegt. Dabei würde sowohl ein Messdruck unter 20 mmHg als auch ein Messdruck über 40 mmHg als außerhalb des Druckbereiches liegend angesehen werden. Bei einem gemessenen Druck innerhalb des zuvor genannten Druckbereiches, also bei einem Messdruck von 20 mmHg bis 40 mmHg, würde hingegen keine Alarmmeldung erfolgen.

Die mittels Pulsoximetrie bestimmte Sauerstoffsättigung ist normalerweise ein prozentualer Wert. Dieser gibt an, wieviel Prozent des roten Blutfarbstoffes Hämoglobin mit Sauerstoff beladen ist. Als unbedenklich und im Normbereich liegend können Werte über 95 % angesehen werden. Entsprechend kann die Untergrenze für die Sauerstoffsättigung aus einem Bereich von 95 % bis 70 %, vorzugsweise 90 % bis 70 %, ausgewählt sein. So kann die Untergrenze für die Sauerstoffsättigung beispielsweise 95 %, 90 %, 85 %, 80 %, 75 % oder 70 % betragen. Insbesondere beträgt die Untergrenze für die Sauerstoffsättigung 90 % oder 85 %.

In einer besonders bevorzugten Ausführungsform des Kompressionstherapiesystems wird für die Untergrenze der Sauerstoffsättigung ein patientenbezogener Wert herangezogen. Dabei ist die Untergrenze für die Sauerstoffsättigung eine vor Beginn oder zu Beginn der Kompressionstherapie von dem Sauerstoffsensor gemessene Sauerstoffsättigung des zu behandelnden beziehungsweise behandelten Körperteils. Dadurch kann die Konstitution des Patienten bei der Überwachung der Kompressionstherapie berücksichtigt werden.

Die Pulsoximetrie erfasst neben der Sauerstoffsättigung des arteriellen Blutes auch den Pulsschlag. Entsprechend kann der Computer vorteilhafterweise weiterhin Mittel zur Ausführung eines Verfahrens zur Überprüfung eines Pulsschlages umfassen. In diesem Verfahren empfängt der Computer während der Kompressionstherapie einen Messwert des Sauerstoffsensors und prüft diesen dahingehend, ob er außerhalb eines vorgegebenen Pulsbereiches liegt. Vorzugsweise wird ein Alarm ausgegeben, wenn die Prüfung ergibt, dass der Messwert des Sauerstoffsensors außerhalb des vorgegebenen Pulsbereiches liegt. Bei dem hierbei verwendeten Messwert kann es sich um denselben Messwert handeln, welcher zur Überprüfung der Kompressionstherapie herangezogen wird. Der Pulsschlag kann Auskunft über den Kreislaufzustand des behandelten Patienten geben und für den Anwender des Kompressionstherapiesystems gleichfalls von Interesse sein. Für die Erfassung des Pulsschlages kann es vorteilhaft sein, wenn der Sauerstoffsensor auch eine Lichtquelle für grünes Licht, insbesondere eine grüne LED, umfasst.

Falls mehrere Sauerstoffsensoren vorhanden sind, kann in dem Verfahren zur Überprüfung des Pulsschlages von jedem Sauerstoffsensor jeweils ein Messwert empfangen werden, wobei die Messwerte der Sauerstoffsensoren zusammengefasst werden, um einen einzelnen, zusammengefassten Sauerstoffsensor-Messwert zu bilden. Der zusammengefasste Sauerstoffsensor-Messwert wird dann dahingehend geprüft, ob er außerhalb des vorgegebenen Pulsbereiches liegt. Vorzugsweise wird der Alarm ausgegeben, wenn die Prüfung ergibt, dass der zusammengefasste Sauerstoffsensor-Messwert außerhalb des vorgegebenen Pulsbereiches liegt. Bevorzugt ist der zusammengefasste Sauerstoffsensor-Messwert ein Mittelwert.

Das Verfahren zur Überprüfung des Pulsschlages könnte bei Vorhandensein von mehreren Sauerstoffsensoren demnach lauten:
Empfangen und Prüfen jeweils eines Messwertes von jedem (der mehreren) Sauerstoffsensor(en) während der Kompressionstherapie,
wobei die Messwerte der Sauerstoffsensoren zusammengefasst werden, um einen einzelnen, zusammengefassten Sauerstoffsensor-Messwert zu bilden,
wobei geprüft wird, ob der zusammengefasste Sauerstoffsensor-Messwert außerhalb eines vorgegebenen Pulsbereiches liegt, und
wobei vorzugsweise ein Alarm ausgegeben wird, wenn die Prüfung ergibt, dass der zusammengefasste Sauerstoffsensor-Messwert außerhalb des vorgegebenen Pulsbereiches liegt.

Alternativ dazu könnte von jedem Sauerstoffsensor jeweils ein Messwert empfangen und im Hinblick auf den vorgegebenen Pulsbereich separat geprüft werden.

Wie der Druckbereich hat auch der Pulsbereich eine Untergrenze und eine Obergrenze. Die Untergrenze des Pulsbereiches kann einen Wert von 40 Schläge/min bis 60 Schläge/min, insbesondere 50 Schläge/min, aufweisen. Die Obergrenze des Pulsbereiches kann einen Wert von 100 Schläge/min bis 200 Schläge/min, insbesondere 150 Schläge/min, aufweisen. Bevorzugt wird also ein Pulsbereich von 50 Schläge/min bis 150 Schläge/min.

Wie bereits erwähnt sind Alarme für den Fall vorgesehen, dass die Messwerte den vorgegebenen Referenzbereichen und Grenzwerten nicht entsprechen. Dabei kann es sinnvoll sein, wenn die Ausgabe der Alarme solange verzögert wird, bis das Ergebnis der Prüfungen einmal oder mehrmals bestätigt wurde. Falsche Alarmmeldungen infolge von nur kurzzeitigen beziehungsweise vorübergehenden Veränderungen von Druck, Sauerstoffsättigung oder Pulsschlag können so vermieden werden. Die hiermit vorgeschlagene Alarmverzögerung knüpft insbesondere an die zuvor genannte Ausführungsform an, in der das Empfangen und Prüfen der Messwerte des Drucksensors und des Sauerstoffsensors automatisch und kontinuierlich oder zumindest automatisch in regelmäßigen Abständen nach Beginn der Kompressionstherapie erfolgt. Typischerweise werden die Alarme visuell und/oder akustisch ausgegeben.

Üblicherweise umfasst der Computer ein Eingabemittel und ein Ausgabemittel. Das Eingabemittel kann dazu dienen, Befehle oder Vorgaben des Benutzers entgegenzunehmen. Das Ausgabemittel kann dazu dienen, die gemessenen Druck-, Sauerstoffsättigungs- und Pulsschlag-Werte anzuzeigen und die Alarme auszugeben. Als sowohl Eingabemittel als auch Ausgabemittel kann ein Berührungsbildschirm (touch screen) von beispielsweise einem Smartphone oder Tablet fungieren. Das Ausgabemittel kann auch ein Lautsprecher sein (akustische Alarme). Jedes Smartphone oder Tablet verfügt heutzutage über einen Berührungsbildschirm und einen Lautsprecher.

Normalerweise umfasst der Computer auch einen Datenträger. Der Datenträger kann dazu dienen, Computerprogramme und die empfangenen Messwerte zu speichern. Dadurch kann der Benutzer den gesamten Verlauf der Kompressionstherapie mit allen empfangenen Messwerten nachvollziehen. Bei dem Datenträger kann es sich um eine Festplatte, ein Halbleiterlaufwerk (solid-state-drive, SSD) oder eine SD-Karte (secure digital memory card) handeln. Denkbar ist zudem, dass die Sensormesswerte von dem Computer des Kompressionstherapiesystems in einem onlinebasierten Datenspeicherdienst (Cloud) oder auf einem lokalen Netzlaufwerk gespeichert werden.

Es wird vorliegend auch ein Verfahren zur Herstellung einer Sensoreinheit für die Überprüfung einer Kompressionstherapie beansprucht. Das Verfahren umfasst die folgenden Schritte:
i. Bereitstellen eines Substrates.
ii. Bereitstellen eines Drucksensors.
iii. Bereitstellen eines pulsoximetrischen Sauerstoffsensors.
iv. Anordnen des Drucksensors und des pulsoximetrischen Sauerstoffsensors auf dem Substrat.

Das Herstellungsverfahren kann gleichfalls Merkmale der zuvor beschriebenen Ausführungsformen der erfindungsgemäßen Sensoreinheit enthalten.

Ergänzend wird die Verwendung einer erfindungsgemäßen Sensoreinheit in allen ihren Ausführungsformen zur Überprüfung einer Kompressionstherapie eines Menschen oder Tieres offenbart. Insbesondere wird die Kompressionstherapie dabei zur Behandlung eines menschlichen Unterschenkels eingesetzt.

Schließlich wird noch der erfindungsgemäße Kit, das erfindungsgemäße Kompressionsmittel (mit der Sensoreinheit) und das erfindungsgemäße System in allen ihren Ausführungsformen zur Anwendung in der Behandlung einer Veneninsuffizienz offenbart. Damit soll auf eine medizinische Indikation der genannten erfindungsgemäßen Gegenstände abgestellt werden. Die Behandlung ist insbesondere für Risikopatienten, zum Beispiel einem Patienten mit einer Veneninsuffizienz und einer arteriellen Verschlusskrankheit, vorgesehen.

### Beispiele und Figuren

Mit den nachfolgend beschriebenen Beispielen und Figuren soll die Erfindung exemplarisch näher erläutert und veranschaulicht werden.

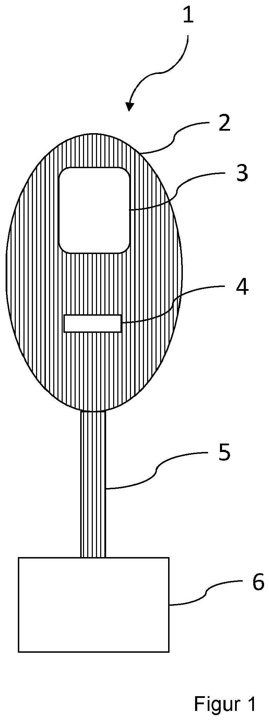

**Figur 1** zeigt eine Sensoreinheit 1 in einer ersten bevorzugten Ausführungsform der Erfindung. Die Sensoreinheit 1 umfasst ein Substrat 2, bei dem es sich um eine bevorzugt flexible Leiterplatte handelt. Auf dem Substrat 2 ist ein Drucksensor 3, zum Beispiel ein piezoelektrischer Drucksensor 3, sowie ein reflexions-pulsoximetrischer Sauerstoffsensor 4 angeordnet. Typischerweise besitzt der Sauerstoffsensor zwei Lichtquellen, jeweils eine für rotes und eine für infrarotes Licht, sowie einen Photodetektor. Ein Konnektor 5 verbindet dann das Substrat 2 mit einer Datenerfassungseinheit 6. Diese hat die Aufgabe, die Messwerte von dem Drucksensor 3 und von dem Sauerstoffsensor 4 zu erfassen und an einen Computer (nicht dargestellt) kabellos, beispielsweise mittels Bluetooth oder NFC, zu übertragen. Hierfür kann die Datenerfassungseinheit 6 einen Datenträger und einen Bluetooth- oder NFC-Sendeempfänger umfassen (nicht dargestellt). Als Computer ist insbesondere ein Smartphone vorgesehen. Der Computer kann die empfangenen Messdaten dann prüfen und den Anwender alarmieren, wenn zum Beispiel der gemessene Druck zu hoch oder die gemessene Sauerstoffsättigung zu niedrig ist.

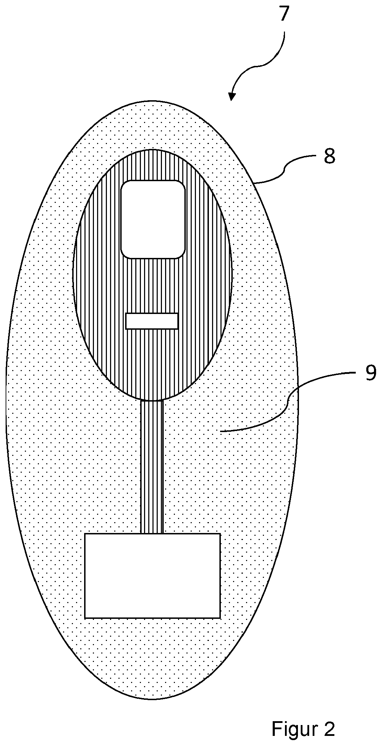

**Figur 2** zeigt eine Sensoreinheit **7** in einer zweiten bevorzugten Ausführungsform der Erfindung. Die Sensoreinheit **7** unterscheidet sich von der Sensoreinheit **1** lediglich dadurch, dass sie in der Art eines Pflasters ausgebildet ist. Das pflasterartige Aussehen ergibt sich durch eine Rückschicht **8** ("backing"), welche auf einer Seite adhäsiv beziehungsweise klebend ausgerüstet ist und an dem Substrat **2**, dem Konnektor **5** und der Datenerfassungseinheit **6** zusätzlich befestigt ist. Mit dem freien, adhäsiven Randbereich **9** der Rückschicht **8** kann die Sensoreinheit am Körper des Patienten angeklebt werden. Die Rückschicht **8** aus **Figur 2** hat eine ovale Form. Die Rückschicht **8** kann aber auch eine andere Form, beispielsweise eine rechteckige Form, haben.

### Anwendungsbeispiel 1

- Zu behandelndes Körperteil: Unterschenkel eines Menschen
- Bestandteile des eingesetzten Kompressionstherapiesystems:
   - Sensoreinheit 1 oder Sensoreinheit 2 aus Figur 1 beziehungsweise Figur 2
   - eine Kompressionsbinde (als Kompressionsmittel, welches losgelöst von der Sensoreinheit bereitgestellt wird)
   - ein Smartphone (als Computer mit einem Datenträger, einem Bluetooth- und NFC-Sendeempfänger, einem Berührungsbildschirm sowie einem Lautsprecher)
- Zeitpunkt der Kontrollmessungen: erfolgt auf Befehl eines Benutzers hin
- Ablauf der Behandlung (in chronologischer Reihenfolge):
   i. **Befestigen der Sensoreinheit** an einer Stelle des Unterschenkels, an der Druck und Sauerstoffsättigung gemessen werden sollen. Für die Befestigung der Sensoreinheit 1 aus Figur 1 kann ein Heftpflaster aus dem medizinischen Bereich, zum Beispiel Omniplast^{®} von der Patentanmelderin, zu Hilfe genommen werden.
   ii. **Umwickeln des Unterschenkels mit der Kompressionsbinde**. Die Sensoreinheit ist dann zwischen Haut und Binde angeordnet. Der Zeitpunkt nach Anlegen der Kompressionsbinde kann als Beginn der Kompressionstherapie angesehen werden.
   iii. **Aktivieren einer auf dem Smartphone installierten Anwendung ("App")**, welche für die Verarbeitung der Sensormesswerte vorgesehen ist. In der Anwendung sind Referenzbereiche und Grenzwerte für Druck, Sauerstoffsättigung und Pulsschlag voreingestellt. Zum Beispiel könnte ein Druckbereich von 20 mmHg bis 40 mmHg, eine Untergrenze für die Sauerstoffsättigung von 85 % und ein Pulsbereich von 50 Schläge/min bis 150 Schläge/min voreingestellt sein. Falls erforderlich kann der Benutzer diese Voreinstellungen entsprechend den spezifischen medizinischen Gegebenheiten individuell anpassen.
   iv. **Starten einer Kontrollmessung** mit Hilfe der Anwendung. Messdaten von dem Drucksensor und dem Sauerstoffsensor werden dabei von der Sensoreinheit an das Smartphone übertragen. Im Ergebnis wird dem Benutzer der Druck angezeigt, welcher von der Kompressionsbinde auf den Unterschenkel ausgeübt wird. Zudem wird dem Benutzer die arterielle Sauerstoffsättigung am Unterschenkel sowie der Pulsschlag angezeigt. Falls einer dieser Parameter nicht mit den eingestellten Bereichen und Grenzwerten übereinstimmt, wird ein entsprechendes Alarmsignal ausgegeben. Damit wird sichergestellt, dass der Benutzer die Abweichung von der Norm in jedem Fall zur Kenntnis nimmt und als mögliche Gefahrensituation wahrnimmt.
   v. **Falls ein Alarmsignal ausgegeben wird, kann eine entsprechende Maßnahme zur Beseitigung des Alarmzustandes erfolgen.** Im Falle eines zu hohen Drucks oder einer zu niedrigen Sauerstoffsättigung kann zum Beispiel die Kompressionsbinde abgenommen und mit geringerem Zug wieder neu angelegt werden. Falls kein Alarmsignal ausgegeben wird, kann die Kompressionstherapie fortgesetzt werden. Der Benutzer hat dann Gewissheit, dass die Kompressionstherapie ordnungsgemäß abläuft.
   vi. **Zu einem späteren, von dem Benutzer frei wählbaren Zeitpunkt kann eine erneute Kontrollmessung durchgeführt werden.** Diese Kontrollmessung muss wie die Kontrollmessung im obigen Schritt iv. vom Benutzer initiiert werden, denn die Datenübertragung und die Datenüberprüfung findet nur auf Abruf statt. Dadurch kann die Menge der übertragenen Messdaten jedoch verringert und in Verbindung mit einem entsprechenden Übertragungsstandard (zum Beispiel NFC) auf eine Batterie für die Sensoren verzichtet werden.

### Anwendungsbeispiel 2

- Zu behandelndes Körperteil: Unterschenkel eines Menschen
- Bestandteile des eingesetzten Kompressionstherapiesystems: Das System ist wie bei dem Anwendungsbeispiel 1 ausgeführt. Als zusätzlichen Bestandteil besitzt die Sensoreinheit eine Batterie für den Druck- und Sauerstoffsensor.
- Zeitpunkt der Kontrollmessungen: automatisch und kontinuierlich
- Ablauf der Behandlung (in chronologischer Reihenfolge):
   i. **Befestigen der Sensoreinheit** (wie bei Anwendungsbeispiel 1).
   ii. **Umwickeln des Unterschenkels mit der Kompressionsbinde** (wie bei Anwendungsbeispiel 1).
   iii. **Aktivieren einer auf dem Smartphone installierten Anwendung** (wie bei Anwendungsbeispiel 1).
   iv. **Messdaten von dem Drucksensor und dem Sauerstoffsensor werden von der Sensoreinheit automatisch und kontinuierlich an das Smartphone übertragen.** Im Ergebnis kann die Anwendung dem Benutzer jederzeit den aktuellen, von der Kompressionsbinde auf den Unterschenkel ausgeübten Druck, die aktuelle arterielle Sauerstoffsättigung am Unterschenkel sowie den aktuellen Pulsschlag anzeigen. Falls einer dieser Parameter nicht mit den eingestellten Bereichen und Grenzwerten übereinstimmt, wird wie im vorangegangenen Anwendungsbeispiel ein entsprechendes Alarmsignal ausgegeben.
   v. **Sobald ein Alarmsignal ausgegeben wird, kann eine entsprechende Maßnahme zur Beseitigung des Alarmzustandes erfolgen** und danach die Kompressionstherapie fortgesetzt werden (wie bei Anwendungsbeispiel 1).
   vi. **Falls es bei der Fortsetzung der Kompressionstherapie erneut zu einem Alarmzustand kommt**, wird dieser durch die automatische und kontinuierliche Datenübertragung und Datenauswertung von der bereits aktiven Anwendung **selbstständig erkannt und an den Benutzer kommuniziert.** Dieses kontinuierliche Monitoring mit automatischer Alarmierung macht die Kompressionstherapie sehr sicher.

### Anwendungsbeispiel 3

- Zu behandelndes Körperteil: Unterschenkel eines Menschen
- Bestandteile des eingesetzten Kompressionstherapiesystems: Das System ist wie bei dem Anwendungsbeispiel 1 ausgeführt.
- Zeitpunkt der Referenz- und Kontrollmessungen: erfolgt auf Befehl eines Benutzers hin
- Ablauf der Behandlung (in chronologischer Reihenfolge):
   i. **Befestigen der Sensoreinheit an einer Stelle des Unterschenkels** (wie bei Anwendungsbeispiel 1).
   ii. **Aktivieren einer auf dem Smartphone installierten Anwendung** (wie bei Anwendungsbeispiel 1).
   iii. **Starten einer Referenzmessung** mit Hilfe der Anwendung. Messdaten von dem Sauerstoffsensor werden dabei von der Sensoreinheit an das Smartphone übertragen. Die Messung dient dem Zweck, den Grenzwert für die Sauerstoffsättigung festzulegen.
   iv. **Umwickeln des Unterschenkels mit der Kompressionsbinde** (wie bei Anwendungsbeispiel 1).
   v. **Starten einer Kontrollmessung** mit Hilfe der Anwendung (wie bei Anwendungsbeispiel 1 mit dem Unterschied, dass die Untergrenze für die Sauerstoffsättigung kein voreingestellter Wert, sondern die in Schritt iii. bestimmte Sauerstoffsättigung vor Beginn der Kompressionstherapie ist).
   vi. **Falls ein Alarmsignal ausgegeben wird, kann eine entsprechende Maßnahme zur Beseitigung des Alarmzustandes erfolgen** und danach die Kompressionstherapie fortgesetzt werden (wie bei Anwendungsbeispiel 1).
   vii. **Zu einem späteren,** von **dem Benutzer frei wählbaren Zeitpunkt kann eine erneute Kontrollmessung durchgeführt werden** (wie bei Anwendungsbeispiel 1).

## Patentansprüche

1. Sensoreinheit (1, 7) für eine Kompressionstherapie zur Überwachung eines auf ein behandeltes Körperteil eines Patienten einwirkenden Drucks sowie zur Überwachung einer Sauerstoffsättigung des arteriellen Bluts in dem behandelten Körperteil des Patienten, umfassend
- ein Substrat (2), wobei es sich bei dem Substrat (2) um eine Leiterplatte handelt,
- einen Drucksensor (3) und
- einen pulsoximetrischen Sauerstoffsensor (4),
wobei der Drucksensor (3) und der Sauerstoffsensor (4) auf dem Substrat (2) angeordnet sind und wobei Messwerte des Drucksensors (3) und des Sauerstoffsensors (4) an einen Computer zur Überprüfung der Kompressionstherapie übertragen werden können.

2. Sensoreinheit nach Anspruch 1, wobei es sich bei dem Substrat (2) um eine flexible Leiterplatte handelt.

3. Sensoreinheit nach Anspruch 1 oder 2, wobei es sich bei dem Drucksensor (3) um einen Dehnungsmessstreifen, einen induktiven Drucksensor oder einen piezoelektrischen Drucksensor handelt.

4. Sensoreinheit nach einem der vorangehenden Ansprüche, wobei der Sauerstoffsensor (4) eine Lichtquelle für rotes Licht, eine Lichtquelle für infrarotes Licht und einen Photodetektor umfasst, wobei die Lichtquellen vorzugsweise Leuchtdioden sind und der Photodetektor vorzugsweise eine Photodiode ist.

5. Sensoreinheit nach einem der vorangehenden Ansprüche, wobei es sich bei dem Sauerstoffsensor (4) um einen reflexions-pulsoximetrischen Sauerstoffsensor handelt.

6. Sensoreinheit nach einem der vorangehenden Ansprüche, wobei es sich bei dem Computer um ein mobiles Gerät, insbesondere ein Smartphone oder ein Tablet, handelt.

7. Sensoreinheit nach einem der vorangehenden Ansprüche, wobei die Übertragung der Messwerte des Drucksensors (3) und des Sauerstoffsensors (4) an den Computer kabellos erfolgt, wobei die Übertragung vorzugsweise über eine Nahfeldkommunikation, Bluetooth oder ein drahtloses lokales Netzwerk erfolgt.

8. Sensoreinheit nach einem der vorangehenden Ansprüche, wobei die Sensoreinheit (1, 7) eine Datenerfassungseinheit (6) umfasst, wobei die Datenerfassungseinheit (6) dafür ausgebildet ist die Messwerte von dem Drucksensor (3) und dem Sauerstoffsensor (4) zu erfassen und an den Computer zu übertragen.

9. Sensoreinheit nach einem der vorangehenden Ansprüche, wobei die Sensoreinheit (1, 7) eine Energiequelle, vorzugsweise eine Batterie, für den Drucksensor und den Sauerstoffsensor umfasst.

10. Sensoreinheit nach einem der Ansprüche 1 bis 8, wobei die Sensoreinheit (1, 7) keine Energiequelle für den Drucksensor und den Sauerstoffsensor umfasst.

11. Sensoreinheit nach einem der vorangehenden Ansprüche, wobei die Sensoreinheit (1, 7) adhäsiv ausgebildet ist und vorzugsweise eine adhäsive Rückschicht (8) umfasst.

12. Kit für eine Kompressionstherapie, umfassend
- ein Kompressionsmittel,
- eine oder mehrere Sensoreinheiten (1, 7) nach einem der vorangehenden Ansprüche,
- optional ein oder mehrere Heftpflaster, und
- optional eine Gebrauchsanweisung.

13. Kompressionsmittel, umfassend eine oder mehrere Sensoreinheiten (1, 7) nach einem der Ansprüche 1 bis 11.

14. System für eine Kompressionstherapie, umfassend
- ein Kompressionsmittel,
- eine Sensoreinheit (1, 7) nach einem der Ansprüche 1 bis 11,
- einen Computer,
wobei der Computer Mittel zur Ausführung eines Verfahrens zur Überprüfung der Kompressionstherapie umfasst, wobei das Verfahren die folgenden Schritte umfasst:
i. Empfangen und Prüfen eines Messwertes des Drucksensors (3) während der Kompressionstherapie,
wobei geprüft wird, ob der Messwert des Drucksensors (3) außerhalb eines vorgegebenen Druckbereiches liegt, und
wobei vorzugsweise ein Alarm ausgegeben wird, wenn die Prüfung ergibt, dass der Messwert des Drucksensors (3) außerhalb des vorgegebenen Druckbereiches liegt,
ii. Empfangen und Prüfen eines Messwertes des Sauerstoffsensors (4) während der Kompressionstherapie,
wobei geprüft wird, ob der Messwert des Sauerstoffsensors (4) eine vorgegebene Untergrenze für eine Sauerstoffsättigung unterschreitet, und
wobei vorzugsweise ein Alarm ausgegeben wird, wenn die Prüfung ergibt, dass der Messwert des Sauerstoffsensors (4) die vorgegebene Untergrenze für die Sauerstoffsättigung unterschreitet.

15. Kit, Kompressionsmittel oder System nach einem der Ansprüche 12 bis 14, wobei das Kompressionsmittel eine Kompressionsbinde, ein Kompressionsschlauch oder ein Kompressionsstrumpf ist.

16. System nach Anspruch 14 oder 15, wobei das Empfangen und Prüfen der Messwerte des Drucksensors (3) und des Sauerstoffsensors (4) automatisch und kontinuierlich oder zumindest automatisch in regelmäßigen Abständen nach Beginn der Kompressionstherapie erfolgt.

17. System nach Anspruch 14 oder 15, wobei das Empfangen und Prüfen der Messwerte des Drucksensors (3) und des Sauerstoffsensors (4) ausschließlich auf Befehl eines Benutzers hin erfolgt.

18. System nach einem der Ansprüche 14 bis 17, wobei
- eine Untergrenze des Druckbereiches einen Wert von 1 mmHg bis 20 mmHg, bevorzugt 10 mmHg bis 20 mmHg und besonders bevorzugt 15 mmHg bis 20 mmHg aufweist, und
- eine Obergrenze des Druckbereiches einen Wert von 40 mmHg bis 200 mmHg, bevorzugt 40 mmHg bis 120 mmHg, mehr bevorzugt 40 mmHg bis 100 mmHg, noch mehr bevorzugt 40 mmHg bis 80 mmHg und besonders bevorzugt 40 mmHg bis 60 mmHg aufweist,
wobei die Untergrenze insbesondere einen Wert von 20 mmHg aufweist und die Obergrenze insbesondere einen Wert von 40 mmHg aufweist.

19. System nach einem der Ansprüche 14 bis 18, wobei die Untergrenze für die Sauerstoffsättigung aus einem Bereich von 95 % bis 70 %, vorzugsweise 90 % bis 70 %, ausgewählt ist, wobei die Untergrenze für die Sauerstoffsättigung insbesondere 90 % oder 85 % beträgt.

20. System nach einem der Ansprüche 14 bis 18, wobei die Untergrenze für die Sauerstoffsättigung eine vor Beginn oder zu Beginn der Kompressionstherapie von dem Sauerstoffsensor (4) gemessene Sauerstoffsättigung ist.

21. System nach einem der Ansprüche 14 bis 20, wobei der Computer Mittel zur Ausführung eines Verfahrens zur Überprüfung eines Pulsschlages umfasst, wobei das Verfahren den folgenden Schritt umfasst:
- Empfangen und Prüfen eines Messwertes des Sauerstoffsensors (4) während der Kompressionstherapie,
wobei geprüft wird, ob der Messwert des Sauerstoffsensors (4) außerhalb eines vorgegebenen Pulsbereiches liegt, und
wobei vorzugsweise ein Alarm ausgegeben wird, wenn die Prüfung ergibt, dass der Messwert des Sauerstoffsensors (4) außerhalb des vorgegebenen Pulsbereiches liegt.

22. System nach Anspruch 21, wobei
- eine Untergrenze des Pulsbereiches einen Wert von 40 Schläge/min bis 60 Schläge/min, insbesondere 50 Schläge/min, aufweist, und
- eine Obergrenze des Pulsbereiches einen Wert von 100 Schläge/min bis 200 Schläge/min, insbesondere 150 Schläge/min, aufweist.

23. Verfahren zur Herstellung einer Sensoreinheit (1, 7) nach einem der Ansprüche 1 bis 11 für die Überprüfung einer Kompressionstherapie, umfassend die Schritte:
i. Bereitstellen eines Substrates (2), wobei es sich bei dem Substrat (2) um eine Leiterplatte handelt,
ii. Bereitstellen eines Drucksensors (3),
iii. Bereitstellen eines pulsoximetrischen Sauerstoffsensors (4),
iv. Anordnen des Drucksensors (3) und des pulsoximetrischen Sauerstoffsensors (4) auf dem Substrat (2).

## Claims

1. Sensor unit (1, 7) for a compression therapy for monitoring a pressure acting on a treated body part of a patient and for monitoring an oxygen saturation of the arterial blood in the treated body part of the patient, comprising
- a substrate (2), the substrate (2) being a printed circuit board,
- a pressure sensor (3) and
- a pulse-oximetric oxygen sensor (4),
wherein the pressure sensor (3) and the oxygen sensor (4) are arranged on the substrate (2), and wherein measurement values from the pressure sensor (3) and the oxygen sensor (4) can be transmitted to a computer for the purpose of examining the compression therapy.

2. Sensor unit according to Claim 1, wherein the substrate (2) is a flexible printed circuit board.

3. Sensor unit according to Claim 1 or 2, wherein the pressure sensor (3) is a strain gauge, an inductive pressure sensor or a piezoelectric pressure sensor.

4. Sensor unit according to any of the preceding claims, wherein the oxygen sensor (4) comprises a light source for red light, a light source for infrared light and a photodetector, wherein the light sources are preferably light-emitting diodes, and the photodetector is preferably a photodiode.

5. Sensor unit according to any of the preceding claims, wherein the oxygen sensor (4) is a reflection pulse-oximetric oxygen sensor.

6. Sensor unit according to any of the preceding claims, wherein the computer is a mobile device, in particular a smartphone or a tablet.

7. Sensor unit according to any of the preceding claims, wherein the transmission of the measurement values from the pressure sensor (3) and the oxygen sensor (4) to the computer is implemented wirelessly, wherein the transmission is preferably implemented by way of near field communication, Bluetooth or a wireless local area network.

8. Sensor unit according to any of the preceding claims, wherein the sensor unit (1, 7) comprises a data acquisition unit (6), wherein the data acquisition unit (6) is designed to acquire the measurement values from the pressure sensor (3) and from the oxygen sensor (4) and transmit said measurement values to the computer.

9. Sensor unit according to any of the preceding claims, wherein the sensor unit (1, 7) comprises a power source, preferably a battery, for the pressure sensor and for the oxygen sensor.

10. Sensor unit according to any of Claims 1 to 8, wherein the sensor unit (1, 7) does not comprise a power source for the pressure sensor and for the oxygen sensor.

11. Sensor unit according to any of the preceding claims, wherein the sensor unit (1, 7) has an adhesive embodiment and preferably comprises an adhesive back layer (8).

12. Kit for a compression therapy, comprising
- a compression means,
- one or more sensor units (1, 7) according to any of the preceding claims,
- optionally one or more plasters, and
- optionally instructions.

13. Compression means, comprising one or more sensor units (1, 7) according to any of Claims 1 to 11.

14. System for a compression therapy, comprising
- a compression means,
- a sensor unit (1, 7) according to any of Claims 1 to 11,
- a computer,
wherein the computer comprises means for carrying out a method for examining the compression therapy, wherein the method comprises the following steps:
i. receiving and checking a measurement value from the pressure sensor (3) during the compression therapy, wherein a check is carried out as to whether the measurement value from the pressure sensor (3) is located outside of a specified pressure range, and preferably wherein an alarm is output if the check yields that the measurement value from the pressure sensor (3) is located outside of the specified pressure range,
ii. receiving and checking a measurement value from the oxygen sensor (4) during the compression therapy, wherein a check is carried out as to whether the measurement value from the oxygen sensor (4) drops below a specified lower limit for an oxygen saturation, and
preferably wherein an alarm is output if the check yields that the measurement value from the oxygen sensor (4) drops below the specified lower limit for the oxygen saturation.

15. Kit, compression means or system according to one of Claims 12 to 14, wherein the compression means is a compression bandage, a compression tubing or a compression stocking.

16. System according to Claim 14 or 15, wherein the measurement values from the pressure sensor (3) and the oxygen sensor (4) are received and checked automatically and continuously or at least automatically at regular intervals following the start of the compression therapy.

17. System according to Claim 14 or 15, wherein the measurement values from the pressure sensor (3) and the oxygen sensor (4) are received and checked only following a user command.

18. System according to any of Claims 14 to 17, wherein
- a lower limit of the pressure range has a value of 1 mmHg to 20 mmHg, preferably 10 mmHg to 20 mmHg and particularly preferably 15 mmHg to 20 mmHg, and
- an upper limit of the pressure range has a value of 40 mmHg to 200 mmHg, preferably 40 mmHg to 120 mmHg, more preferably 40 mmHg to 100 mmHg, even more preferably 40 mmHg to 80 mmHg and particularly preferably 40 mmHg to 60 mmHg,
wherein the lower limit has a value of 20 mmHg in particular, and the upper limit has a value of 40 mmHg in particular.

19. System according to any of Claims 14 to 18, wherein the lower limit for the oxygen saturation is selected from a range of 95% to 70%, preferably 90% to 70%, wherein the lower limit for the oxygen saturation is 90% or 85% in particular.

20. System according to any of Claims 14 to 18, wherein the lower limit for the oxygen saturation is an oxygen saturation measured by the oxygen sensor (4) prior to the start or at the start of the compression therapy.

21. System according to any of Claims 14 to 20, wherein the computer comprises means for carrying out a method for examining a pulse, wherein the method comprises the following step:
- receiving and checking a measurement value from the oxygen sensor (4) during the compression therapy, wherein a check is carried out as to whether the measurement value from the oxygen sensor (4) is located outside of a specified pulse range, and preferably wherein an alarm is output if the check yields that the measurement value from the oxygen sensor (4) is located outside of the specified pulse range.

22. System according to Claim 21, wherein
- a lower limit of the pulse range has a value of 40 beats/min to 60 beats/min, in particular 50 beats/min, and
- an upper limit of the pulse range has a value of 100 beats/min to 200 beats/min, in particular 150 beats/min.

23. Method for producing a sensor unit (1, 7) according to any of Claims 1 to 11 for examining a compression therapy, comprising the following steps:
i. providing a substrate (2), wherein the substrate (2) is a printed circuit board,
ii. providing a pressure sensor (3),
iii. providing a pulse-oximetric oxygen sensor (4),
iv. arranging the pressure sensor (3) and the pulse-oximetric oxygen sensor (4) on the substrate (2).

## Revendications

1. Unité de détection (1, 7) destinée à la thérapie par compression pour surveiller une pression agissant sur une partie du corps traitée d'un patient et pour surveiller une saturation en oxygène du sang artériel dans la partie du corps traitée du patient, ladite unité de compression comprenant
- un substrat (2), le substrat (2) étant une carte de circuit imprimé,
- un capteur de pression (3) et
- un capteur d'oxygène d'oxymétrie de pouls (4),
le capteur de pression (3) et le capteur d'oxygène (4) étant disposés sur le substrat (2) et des valeurs mesurées du capteur de pression (3) et du capteur d'oxygène (4) pouvant être transmises à un ordinateur destiné à vérifier la thérapie par compression.

2. Unité de détection selon la revendication 1, le substrat (2) étant une carte de circuit imprimé flexible.

3. Unité de détection selon la revendication 1 ou 2, le capteur de pression (3) étant une jauge de contrainte, un capteur de pression inductif ou un capteur de pression piézoélectrique.

4. Unité de détection selon l'une des revendications précédentes, le capteur d'oxygène (4) comprenant une source de lumière rouge, une source de lumière infrarouge et un photodétecteur, les sources de lumière étant de préférence des diodes électroluminescentes et le photodétecteur étant de préférence une photodiode.

5. Unité de détection selon l'une des revendications précédentes, le capteur d'oxygène (4) étant un capteur d'oxygène d'oxymétrie de pouls à réflexion.

6. Unité de détection selon l'une des revendications précédentes, l'ordinateur étant un appareil mobile, notamment un smartphone ou une tablette.

7. Unité de détection selon l'une des revendications précédentes, les valeurs mesurées du capteur de pression (3) et du capteur d'oxygène (4) étant transmises sans fil à l'ordinateur, la transmission étant effectuée de préférence par le biais d'une communication en champ proche, d'un Bluetooth ou d'un réseau local sans fil.

8. Unité de détection selon l'une des revendications précédentes, l'unité de détection (1, 7) comprenant une unité d'acquisition de données (6), l'unité d'acquisition de données (6) étant conçue pour acquérir les valeurs de mesure du capteur de pression (3) et du capteur d'oxygène (4) et pour les transmettre à l'ordinateur.

9. Unité de détection selon l'une des revendications précédentes, l'unité de détection (1, 7) comprenant une source d'énergie, de préférence une batterie, destinée au capteur de pression et au capteur d'oxygène.

10. Unité de détection selon l'une des revendications 1 à 8, l'unité de détection (1, 7) ne comprenant pas de source d'énergie destinée au capteur de pression et au capteur d'oxygène.

11. Unité de détection selon l'une des revendications précédentes, l'unité de détection (1, 7) étant conçue pour être adhésive et comprenant de préférence une couche de support adhésive (8).

12. Kit de thérapie par compression, comprenant
- un moyen de compression,
- une ou plusieurs unités de détection (1, 7) selon l'une des revendications précédentes,
- éventuellement un ou plusieurs pansements adhésifs, et
- éventuellement une notice d'utilisation.

13. Moyen de compression, comprenant une ou plusieurs unités de détection (1, 7) selon l'une des revendications 1 à 11.

14. Système de thérapie par compression, comprenant
- un moyen de compression,
- une unité de détection (1, 7) selon l'une des revendications 1 à 11,
- un ordinateur,
l'ordinateur comprenant des moyens de mise en œuvre d'un procédé de vérification de la thérapie par compression, le procédé comprenant les étapes suivantes :
i. recevoir et contrôler une valeur de mesure du capteur de pression (3) pendant la thérapie par compression,
un contrôle étant effectué pour savoir si la valeur de mesure du capteur de pression (3) est située à l'extérieur d'une zone de pression spécifiée, et
une alarme étant de préférence délivrée si le contrôle montre que la valeur de mesure du capteur de pression (3) est située à l'extérieur de la zone de pression spécifiée,
ii. recevoir et contrôler une valeur de mesure du capteur d'oxygène (4) pendant la thérapie par compression,
un contrôle étant effectué pour avoir si la valeur de mesure du capteur d'oxygène (4) devient inférieure à une limite inférieure spécifiée de la saturation en oxygène, et
une alarme étant de préférence délivrée si le contrôle montre que la valeur de mesure du capteur d'oxygène (4) devient inférieure à la limite inférieure spécifiée de la saturation en oxygène.

15. Kit, moyen de compression ou système selon l'une des revendications 12 à 14, le moyen de compression étant un bandage de compression, un tuyau de compression ou un bas de compression.

16. Système selon la revendication 14 ou 15, la réception et le contrôle des valeurs de mesure du capteur de pression (3) et du capteur d'oxygène (4) étant effectués automatiquement et en continu ou au moins automatiquement à intervalles réguliers après le début de la thérapie par compression.

17. Système selon la revendication 14 ou 15, la réception et le contrôle des valeurs de mesure du capteur de pression (3) et du capteur d'oxygène (4) étant effectués exclusivement sur instruction d'un utilisateur.

18. Système selon l'une des revendications 14 à 17,
- une limite inférieure de la zone de pression ayant une valeur de 1 mmHg à 20 mmHg, de préférence de 10 mmHg à 20 mmHg et de manière particulièrement préférée de 15 mmHg à 20 mmHg, et
- une limite supérieure de la zone de pression ayant une valeur de 40 mmHg à 200 mmHg, de préférence de 40 mmHg à 120 mmHg, plus préférablement de 40 mmHg à 100 mmHg, encore plus préférablement de 40 mmHg à 80 mmHg et de manière particulièrement préférée de 40 mmHg à 60 mmHg,
la limite inférieure ayant notamment une valeur de 20 mmHg et la limite supérieure ayant notamment une valeur de 40 mmHg.

19. Système selon l'une des revendications 14 à 18, la limite inférieure de la saturation en oxygène étant choisie dans une plage de 95 % à 70 %, de préférence de 90 % à 70 %, la limite inférieure de la saturation en oxygène étant notamment de 90 % ou 85 %.

20. Système selon l'une des revendications 14 à 18, la limite inférieure de la saturation en oxygène étant une saturation en oxygène mesurée par le capteur d'oxygène (4) avant ou au début de la thérapie par compression.

21. Système selon l'une des revendications 14 à 20, l'ordinateur comprenant des moyens de mise en œuvre d'un procédé de vérification d'un battement de pouls, le procédé comprenant l'étape suivante :
- recevoir et contrôler une valeur de mesure du capteur d'oxygène (4) pendant la thérapie par compression,
une vérification étant effectuée pour savoir si la valeur de mesure du capteur d'oxygène (4) est située à l'extérieur d'une zone de pouls spécifiée, et
une alarme étant de préférence délivrée si le contrôle montre que la valeur de mesure du capteur d'oxygène (4) est située à l'extérieur de la zone de pouls spécifiée.

22. Système selon la revendication 21,
- une limite inférieure de la zone de pouls ayant une valeur de 40 battements/min à 60 battements/min, en particulier de 50 battements/min, et
- une limite supérieure de la zone de pouls ayant une valeur de 100 battements/min à 200 battements/min, notamment 150 battements/min.

23. Procédé de fabrication d'une unité de détection (1, 7) selon l'une des revendications 1 à 11 destinée à vérifier une thérapie par compression, ledit procédé comprenant les étapes suivantes :
i. fournir un substrat (2), le substrat (2) étant une carte de circuit imprimé,
ii. fournir un capteur de pression (3),
iii. fournir un capteur d'oxygène d'oxymétrie de pouls (4),
iv. disposer le capteur de pression (3) et le capteur d'oxygène d'oxymétrie de pouls (4) sur le substrat (2).
